# FASCICULE DE BREVET EUROPEEN

(11) **EP 3 145 448 B1**
(45) Date de publication et mention de la délivrance du brevet: **16.05.2018**
(21) Numéro de dépôt: 15708282.7
(22) Date de dépôt: 22.01.2015
(51) Int. Cl.: A61F 2/24, A61F 2/848

(54) **PROTHÈSE DE VALVE CARDIAQUE MITRALE OU TRICUSPIDE**
PROTHETISCHE MITRAL- ODER TRIKUSPIDALHERZKLAPPE
PROSTHETIC MITRAL OR TRICUSPID HEART VALVE

(30) Priorité: 23.05.2014 FR 1454678
(43) Date de publication de la demande: 29.03.2017
(73) Titulaire: Valmy Holding, 59650 Villeneuve d'Ascq (FR)
(72) Inventeur: MODINE, Thomas, 59110 La Madeleine (FR)
(74) Mandataire: Jeannet, Olivier
(86) Numéro de dépôt international: PCT/IB2015/050498
(87) Numéro de publication internationale: WO 2015/177655

(56) Documents cités:
- WO-A1-2009/132187
- WO-A1-2013/037519
- US-A1- 2012 215 303
- US-A1- 2014 135 908

## Description

La présente invention concerne une prothèse de valve cardiaque mitrale ou tricuspide.

Une pathologie bien connue d'une valve cardiaque, touchant principalement les patients âgés, est la distension de l'anneau valvulaire, cette distension conduisant à une mauvaise coaptation des valvules et donc à la perte d'étanchéité et d'efficacité de la valve.

Il est connu de traiter cette pathologie par annuloplastie, c'est-à-dire par la mise en place d'un anneau prothétique total ou partiel ayant pour but de re-calibrer l'anneau valvulaire natif. Cette technique a certains inconvénients, notamment de ne pas être très adaptée à des patients âgés.

Il est également connu d'implanter, par-dessus la valve native, une prothèse comprenant une armature tubulaire expansible et une valve prothétique montée sur cette armature. L'armature tubulaire, fréquemment dénommée "stent", a une structure maillée, et comprend une portion atriale, c'est-à-dire destinée à prendre place au niveau de l'oreillette d'un coeur, une portion ventriculaire, c'est-à-dire destinée à prendre place au niveau du ventricule d'un coeur, et une portion annulaire, c'est-à-dire destinée à prendre place au niveau de l'anneau valvulaire natif, située entre ces portions atriale et ventriculaire. L'armature peut être auto-expansible (étant notamment en un matériau à mémoire de forme) ou en un matériau expansible au moyen d'un ballonnet. La valve prothétique peut, quant à elle, être en un matériau synthétique ou naturel.

Une telle prothèse est déformable de manière à pouvoir être placée dans un cathéter et est destinée à être déployée depuis ce cathéter au niveau de la valve native à traiter. Elle peut être mise en place par la voie d'abord apicale (c'est-à-dire par la pointe inférieure du coeur) ou par une voie transeptale (valve mitrale) ou jugulaire ou fémorale (valve tricuspide).

Les prothèses de ce type existantes ne donnent pas parfaitement satisfaction, présentant notamment le risque de gêner, lorsqu'elles sont mises en place, le fonctionnement de la valve aortique et des risques de migration et donc d'obstruction de la voie d'éjection ventriculaire. De plus ces prothèses ont une mise en place pas toujours très aisée à opérer, et n'ont pas une forme parfaitement adaptée à celle du site d'implantation.

La présente invention a pour objectif de remédier à ces inconvénients essentiels.

Les publications de demandes de brevet N° WO 2013/037519 A1 et
WO 2009/132187 A1 décrivent des prothèses ne permettant
pas d'atteindre ce but.

La prothèse concernée est définie par les revendications annexées. La partie pré-caractérisante de la revendication 1 définit les caractéristiques qui sont connues par le document N° WO 2013/037519 A1.

Selon l'invention,
- ladite portion ventriculaire présente une large échancrure sur un côté, s'étendant en longueur depuis l'extrémité de cette portion ventriculaire qui est opposée à ladite portion annulaire jusqu'à la partie de cette portion ventriculaire proche de la portion annulaire, cette échancrure s'étendant sur un secteur de la circonférence de la prothèse de l'ordre de 90 à 140°.

L'échancrure interrompt ainsi la structure maillée de l'armature et donne une forme asymétrique à la portion ventriculaire de la prothèse ; cette échancrure est destinée à être tournée vers la paroi du ventricule située à proximité de la valve aortique, zone dite de "continuité mitro-aortique" ou "tricuspido-aortique". Elle permet une absence de structure maillée en regard de cette paroi et donc que la prothèse ne présente aucun risque d'appui excessif contre la paroi du ventricule au droit de la valve aortique, susceptible de gêner le fonctionnement de cette valve aortique.

L'inventeur a cependant conçu que la réduction de la surface d'appui de la prothèse contre la paroi du ventricule, due à l'échancrure, augmentait fortement le risque de migration de la prothèse et donc d'obstruction de la voie d'éjection ventriculaire ; l'inventeur a donc pu concevoir que l'adoption d'une telle échancrure impliquait d'assurer un parfait ancrage de la prothèse au site d'implantation, et qu'il était nécessaire, à cet effet, d'aménager des pointes d'ancrage sur ladite portion atriale et sur ladite portion ventriculaire, en des emplacements proches de ladite portion annulaire, aptes à s'insérer en profondeur dans l'anneau de la valve traitée.

Lorsque la prothèse est mise en place par la voie d'abord apicale, sa portion atriale est tout d'abord déployée et amenée contre la partie atriale de l'anneau valvulaire ; la forme évasée de cette portion atriale est bien adaptée à une large prise d'appui contre cette partie atriale de l'anneau ; une fois ladite portion atriale déployée, les différentes pointes d'ancrage que comprend cette portion atriale sont aptes à venir s'insérer dans ladite partie atriale de l'anneau lorsque le cathéter est reculé afin libérer les autres portions de l'armature. Un bon pré-positionnement de la prothèse vis-à-vis du site d'implantation est ainsi assuré. La poursuite du recul du cathéter réalise le déploiement progressif de la portion ventriculaire ; compte tenu de la forme sphérique ou ovoïde de cette portion ventriculaire, ce déploiement réalise une expansion radiale de la paroi de cette portion ventriculaire, qui amène les différentes pointes d'ancrage que comprend cette portion ventriculaire à s'insérer dans la partie ventriculaire de l'anneau valvulaire, dès le début du déploiement, réalisant ainsi un parfait verrouillage de la prothèse sur l'anneau valvulaire natif. Ce verrouillage est d'autant plus efficient que la forme sphérique ou ovoïde de la portion ventriculaire permet une application étroite de l'armature contre ladite partie ventriculaire de l'anneau valvulaire.

Cette même forme sphérique ou ovoïde permet une prise d'appui de l'armature qui est bien adaptée à la forme du ventricule à proximité de l'anneau valvulaire et qui ne vient pas en conflit avec les structures anatomiques sous-jacentes.

La prothèse ainsi structurée permet donc de parfaitement atteindre l'objectif précité d'obtenir une prothèse ne gênant, lorsqu'elle est en place, le fonctionnement de la valve aortique tout en présentant des risques de migration très faibles et en ayant une mise en place relativement aisée et une forme bien adaptée à celle du site d'implantation.

Il est à noter que la prothèse selon l'invention peut être implantée par une voie d'abord transeptale (valve mitrale) ou jugulaire ou fémorale (valve tricuspide), auquel cas la portion ventriculaire est déployée en premier et la portion atriale est déployée en deuxième, avec des insertions des pointes d'ancrage de la portion ventriculaire puis de la portion atriale dans l'anneau, qui s'opèrent de façon analogue à celle précitée.

De préférence, ladite échancrure s'étend sur un secteur de la circonférence de la prothèse de l'ordre de 120°.

De préférence, lesdites pointes d'ancrage sont sous la forme de griffes courbes se développant vers l'extérieur de la prothèse depuis leurs bases vers leurs extrémités libres pointues.

Les pointes d'ancrage ainsi conformées réalisent un ancrage efficace.

Les pointes d'ancrage sont de préférence présentes sur l'ensemble de la circonférence de la prothèse ; elles sont de préférence régulièrement réparties sur cette circonférence.

Selon une première possibilité, les pointes d'ancrage que comprend ladite portion atriale sont situées en regard des pointes d'ancrage que comprend ladite portion ventriculaire dans le sens longitudinal de la prothèse.

L'ancrage se réalise ainsi par un effet de pince opéré par les différentes paires de pointes d'ancrage, ce qui permet un ancrage très efficace. La particularité de ces pointes est de former alors une sorte de "pince de crabe" du fait que les pointes se dirigent de manière symétrique l'une vers l'autre, permettant de mordre le tissu annulaire et de stabiliser l'armature de la valve. Lors du déploiement de la prothèse avec utilisation de la voie d'abord apicale, les pointes d'ancrage atriales se déploient en premier, permettant d'accrocher le tissu annulaire, puis, dans un deuxième temps, au fur et à mesure de la progression du déploiement, les pointes d'ancrage ventriculaires se déploient à leur tour et permettent de parfaire l'ancrage de la prothèse, avec maintien des pointes d'ancrage en position intra-annulaire.

Ainsi, la structure proposée permet une implantation qui prend appui sur le tissu valvulaire péri-annulaire dans un premier temps (les pointes d'ancrage), qui est suivi par un appui complémentaire sur l'anneau valvulaire. En effet, le système d'accroché en pince permet une accroche du tissu valvulaire et se complète par l'appui intra-annulaire de la structure métallique, et ce, en position mitrale et tricuspide quelque doit l'accès utilisé.

La prothèse peut notamment comprendre six paires de pointes d'ancrage atriales et de pointes d'ancrage ventriculaires.

Selon une deuxième possibilité, à l'état déployé de la prothèse :
- chaque pointe d'ancrage présente une portion de base sensiblement rectiligne, faisant saillie de la portion de la prothèse selon une direction formant un angle de 35 à 55° par rapport à l'axe longitudinal de la prothèse, et une portion d'extrémité libre qui est coudée par rapport à ladite portion de base et s'oriente selon une direction sensiblement circonférentielle de la prothèse ;
- la portion d'extrémité libre d'une pointe d'ancrage atriale est orientée en direction de la portion d'extrémité libre d'une pointe d'ancrage ventriculaire adjacente, proche dans la direction circonférentielle mais décalée angulairement par rapport à cette pointe d'ancrage atriale ; réciproquement, la portion d'extrémité libre d'une pointe d'ancrage ventriculaire est orientée en direction de la portion d'extrémité libre d'une pointe d'ancrage atriale adjacente, proche dans la direction circonférentielle mais décalée angulairement par rapport à cette pointe d'ancrage ventriculaire.

Cette forme de pointes d'ancrage permet un ancrage efficace de la prothèse à l'anneau de la valve traitée.

Il sera compris que l'expression "pointe d'ancrage atriale" désigne une pointe d'ancrage présente sur ladite portion atriale et que l'expression "pointe d'ancrage ventriculaire" désigne une pointe d'ancrage présente sur ladite portion ventriculaire.

De préférence, chaque portion de base fait saillie selon une direction formant un angle de l'ordre de 45° par rapport à l'axe longitudinal de la prothèse.

De préférence, sur chaque pointe, la longueur de la portion de base est de l'ordre de deux à trois fois celle de la portion d'extrémité libre.

De préférence, pour une prothèse de traitement d'une valve mitrale, les pointes d'ancrage sont réparties de la manière suivante sur la circonférence de l'armature de la prothèse : une pointe située sur la zone de cette armature destinée à se trouver au niveau du trigone antérieur après implantation, une pointe située sur la zone de l'armature destinée à se trouver au niveau du trigone postérieur après implantation,une pointe située sur la zone de l'armature, destinée à se trouver après implantation au niveau de la commissure antérieure de la valve mitrale, une pointe située sur la zone de l'armature destinée à se trouver après implantation, au niveau de la commissure postérieure de la valve mitrale, une pointe située sur la zone de l'armature, destinée à se trouver après implantation, au milieu de l'anneau postérieur de la valve mitrale et une pointe située sur la zone de l'armature, destinée à se trouver après implantation, au milieu de l'anneau antérieur de la valve mitrale.

De préférence, la structure maillée de l'armature comprend, au niveau desdites portions atriale et annulaire, et au niveau de la partie de la portion ventriculaire comprenant lesdites pointes d'ancrage, des mailles en forme de losange reliées les unes aux autres par leurs angles. Lorsque les pointes d'ancrage sont selon la première possibilité précitée, elles peuvent avoir leurs bases reliées aux portions des mailles qui forment les angles opposés de ces mailles dans la direction atriale-ventriculaire de la prothèse, ces pointes d'ancrage faisant saillie depuis ces portions des mailles.

Cette structure maillée permet à l'armature de prendre un appui efficace contre les parties correspondantes de l'anneau valvulaire et d'assurer une bonne insertion desdites pointes d'ancrage dans cet anneau.

De préférence, la structure maillée de l'armature comprend, au niveau de la partie de la portion ventriculaire dépourvue de pointes d'ancrage, des mailles en forme de gouttes d'eau, avec deux mailles adjacentes disposées de façon têtes bêches l'une par rapport à l'autre.

Cette structure maillée permet à l'armature d'avoir une souplesse importante au niveau de ladite partie de portion ventriculaire, qui est adaptée à la prise d'appui contre la paroi du ventricule d'un coeur.

De préférence, lorsque la prothèse est destinée à traiter une valve mitrale, la portion atriale et la portion annulaire de la prothèse présentent une section transversale non circulaire, en forme de "D à angles et bords arrondis".

Il sera compris que l'expression "section transversale" fait référence à la section de la prothèse perpendiculairement à l'axe longitudinal de la prothèse.

Ces portions atriale et annulaire présentent ainsi une forme bien adaptée à celles de l'oreillette à proximité de l'anneau d'une valve mitrale et de cet anneau.

De préférence, la portion atriale de la prothèse comporte une paroi étanche qui recouvre ses mailles. Cette paroi étanche, notamment en polyéthylène téréphtalate, forme une membrane ou chemise qui permet d'augmenter la surface de contact de cette portion et de la paroi cardiaque afin de diminuer les fuites paravalvulaires, c'est-à-dire entre la prothèse et la paroi cardiaque.

L'invention sera bien comprise, et d'autres caractéristiques et avantages de celle-ci apparaîtront, en référence au dessin schématique annexé, qui représente, à titre d'exemples non limitatifs, deux formes de réalisation possibles de la prothèse concernée.
La figure 1 en est une vue de côté selon une première forme de réalisation, avec la valve prothétique que comprend cette prothèse en position d'ouverture ;
la figure 2a est une vue agrandie d'une portion de la prothèse telle que montrée sur la figure 1, comprenant des griffes d'ancrage ;
la figure 2b est une vue de cette même portion, en coupe, selon un angle perpendiculaire à la vue selon la figure 2a ;
la figure 3 est une vue de la prothèse par son extrémité ventriculaire, légèrement désaxée, avec la valve prothétique en position d'ouverture ;
la figure 4 en est une vue par son extrémité ventriculaire, selon l'axe de cette prothèse, avec la valve prothétique en position d'ouverture ;
la figure 5 en est une vue similaire à la figure 1, avec la valve prothétique en position de fermeture ;
la figure 6 en est une vue par son extrémité atriale, légèrement désaxée, avec la valve prothétique en position de fermeture ;
la figure 7 est une vue en perspective de l'armature que comprend la prothèse selon une deuxième forme de réalisation ;
la figure 8 en est une vue de côté ;
la figure 9 en est une vue en coupe selon la ligne IX-IX de la figure 8, et
la figure 10 est une vue de la forme que présente le conduit délimité par la portion annulaire de la prothèse, tel que visible sur la figure 9.

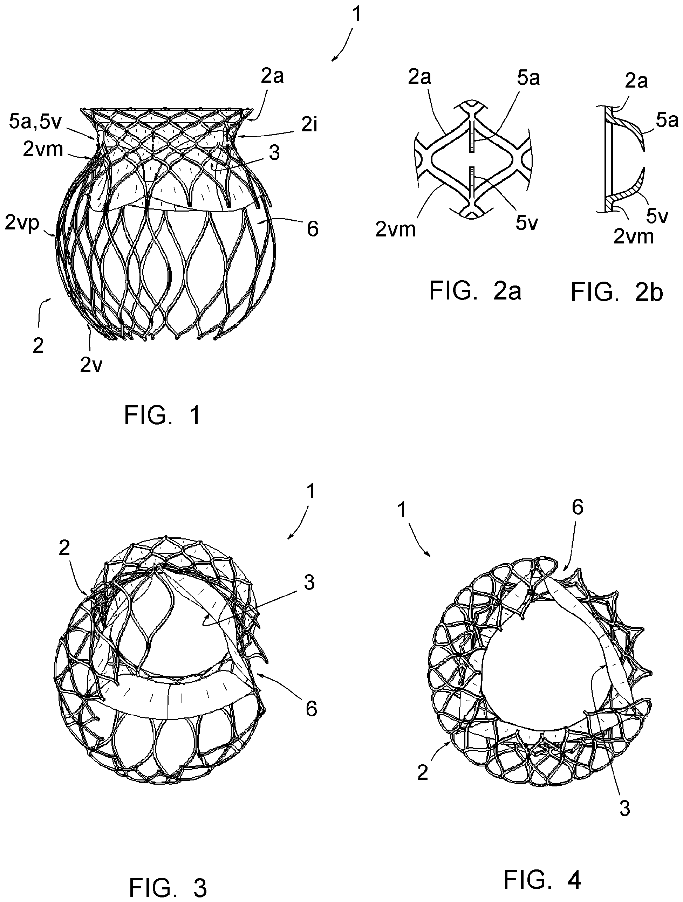

Les figures 1 à 6 représentent une prothèse 1 de valve cardiaque mitrale ou tricuspide, comprenant une armature tubulaire expansible 2 et une valve prothétique 3 montée sur cette armature.

L'armature 2 a structure maillée formée de filaments élastiquement déformables, lui permettant de prendre la forme déployée représentée sur les figures et une forme contractée (non représenté) dans laquelle elle peut être contenue dans un cathéter d'introduction dans un coeur. L'armature 2 peut notamment être réalisée, par une technique connue, en alliage à mémoire de forme, notamment en alliage de nickel et de titane connu sous la dénomination Nitinol.

L'armature 2 comprend une portion atriale 2a, c'est-à-dire destinée à prendre place au niveau de l'oreillette d'un coeur, une portion ventriculaire 2v, c'est-à-dire destinée à prendre place au niveau du ventricule d'un coeur, et une portion annulaire intermédiaire 2i, située entre ces portions atriale 2a et ventriculaire 2v, destinée à prendre place au niveau de l'anneau valvulaire natif.

A l'état de déploiement représenté, la portion atriale 2a présente une forme conique dont la section va en augmentant depuis la portion annulaire 2i, et ayant un angle au sommet de l'ordre de 120°. Elle est formée par des mailles en losange reliées les unes aux autres par leurs angles, et comprend six griffes d'ancrage 5a régulièrement réparties sur sa circonférence. Comme plus particulièrement visible sur les figures 2a et 2b, ces griffes 5a ont leurs bases reliées aux portions des mailles qui forment les angles des mailles situés sur le côté atrial de la prothèse 1, et font saillie depuis ces portions des mailles, vers le côté ventriculaire de la prothèse. Elles sont courbes et se développent vers l'extérieur de la prothèse 1 depuis ces bases vers leurs extrémités libres pointues.

La portion annulaire intermédiaire 2i est formée des mêmes mailles en losange que la portion atriale 2a, dans la continuité de ces mailles.

La portion ventriculaire 2v présente une forme sensiblement ovoïde. Elle comprend une partie marginale 2vm proche de la portion annulaire intermédiaire 2i, raccordée à cette dernière, comportant des griffes d'ancrage 5v, et une partie principale 2vp raccordée à cette partie marginale du côté opposé à la portion 2i, présentant une large échancrure 6.

La partie marginale 2vm est formée des mêmes mailles en losange que la portion atriale 2a et la portion intermédiaire 2i, dans la continuité de ces mailles. Les griffes 5v qu'elle comprend sont situées en regard des griffes 5a et ont une structure identique à celle de ces griffes, ayant leurs bases reliées aux portions des mailles qui forment les angles des mailles situés sur le côté ventriculaire de la prothèse 1, faisant saillie depuis ces portions des mailles, étant courbes et se développant vers l'extérieur de la prothèse 1 depuis ces bases vers leurs extrémités libres pointues.

La partie principale 2vp est formée de mailles en gouttes d'eau, disposées de façon têtes bêches d'une maille à une maille adjacente.

L'échancrure 6 s'étend en longueur depuis l'extrémité de la portion ventriculaire 2v opposée à la portion annulaire 2i jusqu'à sa partie marginale 2vm, et s'étend sur un secteur de l'ordre de 120°. Elle donne ainsi une forme asymétrique à la portion ventriculaire 2v.

La valve prothétique 2, quant à elle est de type connu, réalisée à partir d'un matériau synthétique ou naturel (tel que du péricarde de porc). Dans l'exemple représenté, elle comprend trois valvules.

En pratique, la prothèse 1 est contractée et placée dans un cathéter d'introduction dans un coeur. Ce cathéter comprend des marqueurs radio-opaques permettant de visualiser l'orientation angulaire de ce cathéter, et donc de la prothèse 1, afin d'orienter la prothèse angulairement avant le déploiement de celle-ci.

Le cathéter peut être introduit dans un coeur par la voie d'abord apicale et est orienté angulairement de manière à orienter la prothèse 1 de telle sorte que l'échancrure 6 soit tournée vers la paroi du ventricule longeant la valve aortique, zone dite de continuité mitro-aortique.

La portion atriale 2a de la prothèse 1 est tout d'abord déployée et amenée contre la partie atriale de l'anneau valvulaire ; la forme conique de cette portion atriale 2a est bien adaptée à une large prise d'appui contre cette partie atriale de l'anneau ; une fois ladite portion atriale déployée, les différentes pointes d'ancrage 5a sont orientées pour venir s'insérer dans ladite partie atriale de l'anneau lorsque le cathéter est reculé afin libérer les autres portions de l'armature 2. Un bon pré-positionnement de la prothèse 1 vis-à-vis du site d'implantation est ainsi assuré.

La poursuite du recul du cathéter réalise le déploiement progressif de la portion ventriculaire 2v ; compte tenu de la forme ovoïde de cette portion ventriculaire, ce déploiement réalise une expansion radiale de la paroi de cette portion ventriculaire 2v, qui amène les différentes pointes d'ancrage 5v à s'insérer dans la partie ventriculaire de l'anneau valvulaire, dès le début du déploiement, réalisant ainsi un parfait verrouillage de la prothèse 1 sur l'anneau valvulaire natif. Ce verrouillage est d'autant plus efficient que la forme ovoïde de la portion ventriculaire 2v permet une application étroite de l'armature 2 contre ladite partie ventriculaire de l'anneau valvulaire.

Cette même forme ovoïde permet une prise d'appui de l'armature 2 qui est bien adaptée à la forme du ventricule et qui ne vient pas en conflit avec les structures anatomiques sous-jacentes.

L'échancrure 6 interrompt la structure maillée de l'armature 2 et permet que la prothèse selon l'invention ne présente aucun risque de gêner le bon fonctionnement de la valve aortique ou d'obstruer la chambre de chasse ventriculaire.

Il est à noter que la prothèse selon l'invention pourrait être implantée par une voie d'abord transeptale (valve mitrale) ou jugulaire (valve tricuspide), auquel cas la portion ventriculaire 2v est déployée en premier et la portion atriale 2a est déployée en deuxième, avec des insertions des pointes d'ancrage 5v, 5a de la portion ventriculaire puis de la portion atriale dans l'anneau qui s'opèrent de manière analogue à celle précitée.

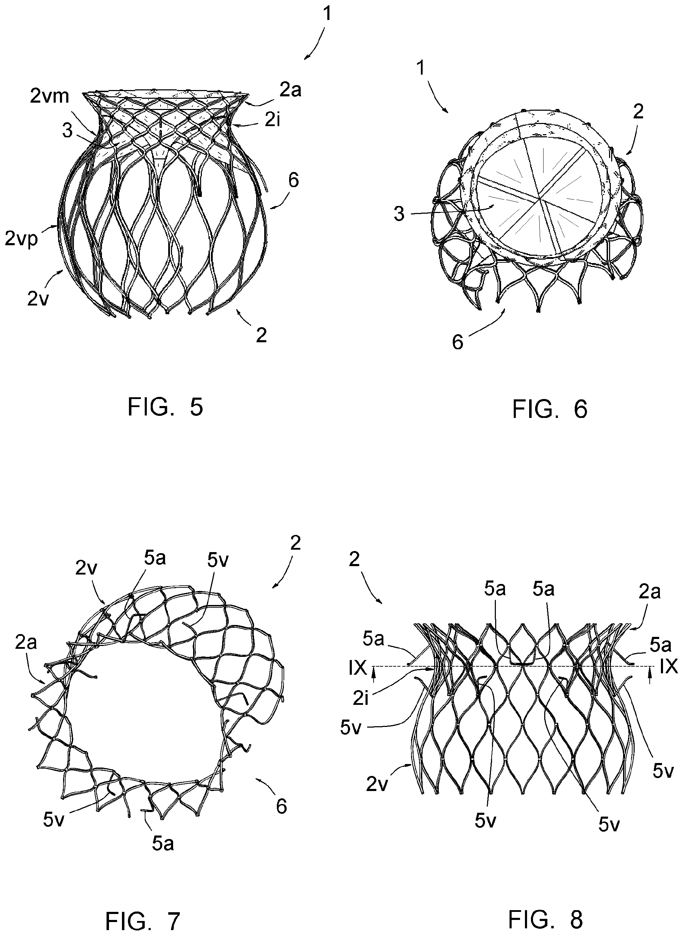

Les figures 7 à 9 représentent, sous différents angles, une deuxième forme de réalisation de l'armature 2 de la prothèse (la valve prothétique n'est pas représentée), destinée au traitement d'une valve mitrale. Par simplification, les parties ou éléments déjà décrits, qui se retrouvent dans cette 2e forme de réalisation, sont désignés par les mêmes références numériques.

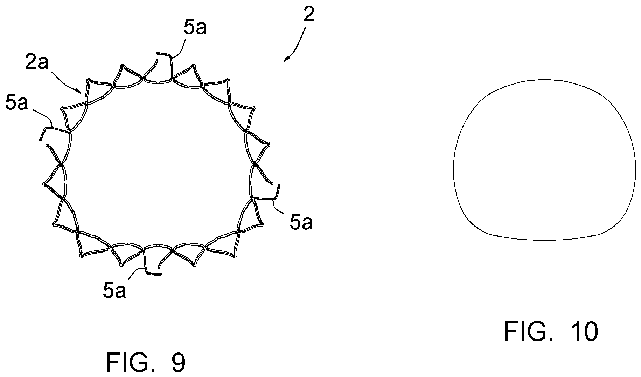

Dans ce cas, la portion atriale 2a n'est pas conique en ce sens qu'elle présente une section transversale non circulaire mais en forme de "D à angles et bords arrondis" visible sur la figure 9 et représentée schématiquement sur la figure 10. En référence à cette figure 10, il apparaît que cette section présente une portion postérieure arrondie, une portion antérieure faiblement arrondie et deux portions latérales arrondies, ces différentes portions étant reliées les unes aux autres par des portions intermédiaires de plus faible rayon de courbure qu'elles. Il sera compris que les termes "postérieure" et "antérieure" ci-dessus font référence respectivement à la portion de la prothèse située sur le côté postérieur de l'anneau valvulaire mitral et sur le côté antérieur de ce même anneau après implantation.

De la même façon que sur l'armature selon la première forme de réalisation, la portion atriale 2a s'évase vers l'extérieur de la prothèse, ayant une section allant en augmentant, depuis ladite portion annulaire 2i vers l'extrémité de cette portion atriale 2a opposée à cette portion annulaire 2i.

Les portions annulaire 2i et ventriculaire 2v présentent des formes similaires à celles de la prothèse selon la première forme de réalisation, sinon que la portion ventriculaire 2v est moins refermée à son extrémité opposée à la portion annulaire 2i, étant tronquée par rapport à celle selon la première forme de réalisation.

L'armature 2 selon la deuxième forme de réalisation diffère de celles selon la première forme de réalisation par la forme des pointes d'ancrage 5a, 5v. En effet,
chaque pointe d'ancrage 5a, 5v présente une portion de base reliée à la portion 2a, 2v correspondante de l'armature 2 et une portion d'extrémité libre qui est coudée par rapport à ladite portion de base. Cette portion de base est sensiblement rectiligne et fait saillie de la portion 2a, 2v selon une direction formant un angle de l'ordre de 45° par rapport à l'axe longitudinal de l'armature (voir particulièrement la figure 8) ; elle a une longueur de l'ordre de deux à trois fois celle de la portion d'extrémité libre. Cette dernière est coudée par rapport à la portion de base et s'oriente selon une direction sensiblement circonférentielle de la prothèse.

Il apparaît que les pointes d'ancrage 5a, 5v sont réparties de la manière suivante sur la circonférence de l'armature 2 : une pointe située sur la zone de cette armature se trouvant au niveau du trigone antérieur après implantation, une pointe située sur la zone de l'armature 2 se trouvant au niveau du trigone postérieur après implantation, une pointe située sur la zone de l'armature 2 se trouvant, après implantation, au niveau de la commissure antérieure de la valve mitrale, une pointe située sur la zone de l'armature 2 se trouvant, après implantation, au niveau de la commissure postérieure de la valve mitrale, une pointe située sur la zone de l'armature 2 se trouvant, après implantation, au milieu de l'anneau postérieur de la valve mitrale et une pointe située sur la zone de l'armature 2 se trouvant, après implantation, au milieu de l'anneau antérieur de la valve mitrale.

Il apparaît également que la portion d'extrémité libre d'une pointe d'ancrage atriale 5a est orientée en direction de la portion d'extrémité libre d'une pointe d'ancrage ventriculaire 5v adjacente, proche dans la direction circonférentielle mais décalée angulairement par rapport à cette pointe d'ancrage atriale 5a ; réciproquement, la portion d'extrémité libre d'une pointe d'ancrage ventriculaire 5v est orientée en direction de la portion d'extrémité libre d'une pointe d'ancrage atriale 5a adjacente, proche dans la direction circonférentielle mais décalée angulairement par rapport à cette pointe d'ancrage ventriculaire.

En considérant particulièrement la figure 8, il faut comprendre que, sur cette vue de côté, la portion antérieure de la prothèse (c'est-à-dire celle la plus proche de l'observateur) se voit de façon superposée à la portion postérieure de la prothèse, faisant que les deux pointes atriales 5a centrales apparaissent proches l'une de l'autre sur cette figure 8 alors qu'elles se situent l'une sur le côté antérieur de la prothèse et l'autre sur le côté postérieur de cette prothèse ; il en est de même des deux pointes ventriculaires 5v centrales, celle située le plus à gauche sur cette figure 8 étant associée à la pointe atriale 5a centrale située le plus à droite, et la pointe atriale 5a située le plus à gauche étant associée à la pointe ventriculaire 5v située le plus à droite.

L'invention fournit ainsi une prothèse 1 de valve cardiaque mitrale ou tricuspide, présentant, par rapport aux prothèses homologues de la technique antérieure, les avantages déterminants de ne pas gêner, lorsqu'elle est mise en place, le fonctionnement de la valve aortique et de présenter un faible risque de migration, et donc d'obstruction de la voie d'éjection ventriculaire. De plus cette prothèse a une mise en place relativement aisée à opérer, et a une forme parfaitement adaptée à celle du site d'implantation.

L'invention a été décrite ci-dessus en référence à des formes de réalisation données à titre d'exemple. Il va de soi qu'elle n'est pas limitée à ces formes de réalisation mais qu'elle s'étend à toutes les autres formes de réalisation couvertes par les revendications ci-annexées.

## Revendications

1. - Prothèse (1) de valve cardiaque mitrale ou tricuspide, comprenant une armature tubulaire expansible (2) à structure maillée et une valve prothétique (3) montée sur cette armature, ladite armature (2) comprenant une portion atriale (2a), une portion ventriculaire (2v) et une portion annulaire (2i) située entre ces portions atriale et ventriculaire ;
- ladite portion atriale (2a) va en s'évasant vers l'extérieur de la prothèse depuis ladite portion annulaire (2i) vers l'extrémité de cette portion atriale opposée à cette portion annulaire, et comprend, du côté de ladite portion annulaire (2i), sur au moins une partie de sa circonférence, des pointes d'ancrage (5a) faisant saillie de sa face extérieure ; et
- ladite portion ventriculaire (2v) présente une forme sensiblement sphérique ou ovoïde et comprend, du côté de ladite portion annulaire (2i), sur au moins une partie de sa circonférence, des pointes d'ancrage (5v) faisant saillie de sa face extérieure.
**caractérisée en ce que** :
- ladite portion ventriculaire (2v) présente une large échancrure (6) sur un côté, s'étendant en longueur depuis l'extrémité de cette portion ventriculaire (2v) qui est opposée à ladite portion annulaire (2i) jusqu'à la partie (2vm) de cette portion ventriculaire (2v) proche de la portion annulaire (2i), cette échancrure (6) s'étendant sur un secteur de la circonférence de la prothèse (1) de l'ordre de 90 à 140° ;

2. - Prothèse (1) selon la revendication 1, **caractérisée en ce que** ladite échancrure s'étend sur un secteur de la circonférence de la prothèse de l'ordre de 120°.

3. - Prothèse (1) selon la revendication 1 ou la revendication 2, **caractérisée en ce que** lesdites pointes d'ancrage (5a, 5v) sont sous la forme de griffes courbes se développant vers l'extérieur de la prothèse depuis leurs bases vers leurs extrémités libres pointues.

4. - Prothèse (1) selon l'une des revendications 1 à 3, **caractérisée en ce que** les pointes d'ancrage (5a, 5v) sont présentes sur l'ensemble de sa circonférence.

5. - Prothèse (1) selon la revendication 4, **caractérisée en ce que** les pointes d'ancrage (5a, 5v) sont régulièrement réparties sur l'ensemble de sa circonférence.

6. - Prothèse selon l'une des revendications 1 à 5, **caractérisée en ce que** les pointes d'ancrage (5a) que comprend ladite portion atriale (2a) sont situées en regard des pointes d'ancrage (5v) que comprend ladite portion ventriculaire (2v).

7. - Prothèse (1) selon l'une des revendications 1 à 6, **caractérisée en ce qu'**elle comprend six paires de pointes d'ancrage atriales (5a) et de pointes d'ancrage ventriculaires (5v).

8. - Prothèse (1) selon la revendication 1, **caractérisée en ce qu'**à l'état déployée de la prothèse :
- chaque pointe d'ancrage (5a, 5v) présente une portion de base sensiblement rectiligne, faisant saillie de la portion de la prothèse selon une direction formant un angle de 35 à 55° par rapport à l'axe longitudinal de la prothèse, et une portion d'extrémité libre qui est coudée par rapport à ladite portion de base et s'oriente selon une direction sensiblement circonférentielle de la prothèse ;
- la portion d'extrémité libre d'une pointe d'ancrage atriale (5a) est orientée en direction de la portion d'extrémité libre d'une pointe d'ancrage ventriculaire (5v) adjacente, proche dans la direction circonférentielle mais décalée angulairement par rapport à cette pointe d'ancrage atriale (5a) ; réciproquement, la portion d'extrémité libre d'une pointe d'ancrage ventriculaire (5v) est orientée en direction de la portion d'extrémité libre d'une pointe d'ancrage atriale (5a) adjacente, proche dans la direction circonférentielle mais décalée angulairement par rapport à cette pointe d'ancrage ventriculaire.

9. - Prothèse (1) selon la revendication 8, **caractérisée en ce que** chaque portion de base fait saillie selon une direction formant un angle de l'ordre de 45° par rapport à l'axe longitudinal de la prothèse.

10. - Prothèse (1) selon la revendication 8 ou la revendication 9, **caractérisée en ce que** sur chaque pointe, la longueur de la portion de base est de l'ordre de deux à trois fois celle de la portion d'extrémité libre.

11. - Prothèse (1) selon l'une des revendications 8 à 10, de traitement d'une valve mitrale, **caractérisée en ce que** les pointes d'ancrage (5a, 5v) sont réparties de 30 la manière suivante sur la circonférence de l'armature (2) de la prothèse : une pointe située sur la zone de cette armature destinée à se trouver au niveau du trigone antérieur après implantation, une pointe située sur la zone de l'armature (2) destinée à se trouver au niveau du trigone postérieur après implantation, une pointe située sur la zone de l'armature (2) destinée à se trouver, après implantation, au niveau de la commissure antérieure de la valve mitrale, une pointe située sur la zone de l'armature (2) destinée à se trouver, après implantation, au niveau de la commissure postérieure de la valve mitrale, une pointe située sur la zone de l'armature (2) destinée à se trouver, après implantation, au milieu de l'anneau postérieur de la valve mitrale et une pointe située sur la zone de l'armature (2) destinée à se trouver, après implantation, au milieu de l'anneau antérieur de la valve mitrale.

12. - Prothèse (1) selon l'une des revendications 1 à 11, **caractérisée en ce que** la structure maillée de l'armature (2) comprend, au niveau desdites portions atriale (2a) et annulaire (2i), et au niveau de la partie de la portion ventriculaire (2v) comprenant lesdites pointes d'ancrage (5v), des mailles en forme de losange reliées les unes aux autres par leurs angles.

13. - Prothèse (1) selon la revendication 12 rattachée à l'une des revendications 3 à 7, **caractérisée en ce que** les pointes d'ancrage (5a, 5v) ont leurs bases reliées aux portions des mailles qui forment les angles opposés de ces mailles dans la direction atriale-ventriculaire de la prothèse, ces pointes d'ancrage (5a, 5v) faisant saillie depuis ces portions des mailles.

14. - Prothèse (1) selon l'une des revendications 1 à 13, **caractérisée en ce que** la structure maillée de l'armature (2) comprend, au niveau de la partie de la portion ventriculaire (2v) dépourvue de pointes d'ancrage (5v), des mailles en forme de gouttes d'eau, deux mailles adjacentes étant disposées de façon têtes bêches l'une par rapport à l'autre.

15. - Prothèse (1) selon l'une des revendications 1 à 14, destinée à traiter une valve mitrale, **caractérisée en ce que** sa portion atriale (2a) et sa portion annulaire (2i) présentent une section non circulaire, en forme de "D à angles et bords arrondis".

16. - Prothèse (1) selon l'une des revendications 1 à 15, **caractérisée en ce que** sa portion atriale (2a) comporte une paroi étanche qui recouvre ses mailles.

## Patentansprüche

1. Mitral- oder Trikuspidal-Herzklappenprothese (1), umfassend eine expandierbare röhrenförmige Armatur (2) mit einer Maschenstruktur und eine prothetische Klappe (3), die an dieser Armatur montiert ist, wobei die Armatur (2) einen Atrialbereich (2a), einen Ventrikularbereich (2v) und einen ringförmigen Bereich (2i) umfasst, der zwischen diesem Atrial- und dem Ventrikularbereich angeordnet ist;
- wobei der Atrialbereich (2a) unter Aufweitung zur Außenseite der Prothese hin von dem ringförmigen Bereich (2i) zu dem Ende dieses Atrialbereichs hin verläuft, der diesem ringförmigen Bereich entgegengesetzt ist, und an der Seite des ringförmigen Bereichs (2i) auf wenigstens einem Teil seines Umfangs Verankerungsspitzen (5a) umfasst, die von seiner Außenoberfläche vorstehen; und
- wobei der Ventrikularbereich (2v) eine im Wesentlichen kugelförmige oder eiförmige Gestalt aufweist und an der Seite des ringförmigen Bereichs (2i) auf wenigstens einem Teil seines Umfangs Verankerungsspitzen (5v) umfasst, die von seiner Außenoberfläche vorstehen,
**dadurch gekennzeichnet, dass**:
- der Ventrikularbereich (2v) eine große Einbuchtung (6) an einer Seite aufweist, die sich in der Länge von dem Ende dieses Ventrikularbereichs (2v), das dem ringförmigen Bereich (2i) entgegengesetzt ist, bis zu dem Teil (2vm) dieses Ventrikularbereichs (2v) erstreckt, das nahe dem ringförmigen Bereich (2i) ist, wobei sich diese Einbuchtung (6) über einen Sektor des Umfangs der Prothese (1) in der Größenordnung von 90 bis 140° erstreckt.

2. Prothese (1) nach Anspruch 1, **dadurch gekennzeichnet, dass** sich die Einbuchtung über einen Sektor des Umfangs der Prothese in der Größenordnung von 120° erstreckt.

3. Prothese (1) nach Anspruch 1 oder Anspruch 2, **dadurch gekennzeichnet, dass** die Verankerungsspitzen (5a, 5v) in der Form von gekrümmten Krallen sind, die sich ausgehend von ihren Basen in Richtung ihrer freien Spitzenenden zur Außenseite der Prothese hin entwickeln.

4. Prothese (1) nach einem der Ansprüche 1 bis 3, **dadurch gekennzeichnet, dass** die Verankerungsspitzen (5a, 5v) auf ihrem gesamten Umfang vorhanden sind.

5. Prothese (1) nach Anspruch 4, **dadurch gekennzeichnet, dass** die Verankerungsspitzen (5a, 5v) gleichmäßig über ihren gesamten Umfang verteilt sind.

6. Prothese nach einem der Ansprüche 1 bis 5, **dadurch gekennzeichnet, dass** die Verankerungsspitzen (5a), die der Atrialbereich (2a) umfasst, gegenüber den Verankerungsspitzen (5v) angeordnet sind, die der Ventrikularbereich (2v) umfasst.

7. Prothese (1) nach einem der Ansprüche 1 bis 6, **dadurch gekennzeichnet, dass** sie sechs Paare von Atrialverankerungsspitzen (5a) und von Ventrikularverankerungsspitzen (5v) umfasst.

8. Prothese (1) nach Anspruch 1, **dadurch gekennzeichnet, dass** im Einsatzzustand der Prothese:
- jede Verankerungsspitze (5a, 5v) einen im Wesentlichen geradlinigen Basisbereich aufweist, der von dem Bereich der Prothese entlang einer Richtung vorsteht, die einen Winkel von 35 bis 55° bezüglich der Längsachse der Prothese bildet, sowie einen freien Endbereich, der bezüglich des Basisbereichs abgewinkelt ist und sich im Wesentlichen entlang einer Umfangsrichtung der Prothese orientiert;
- der freie Endbereich eine Atrialverankerungsspitze (5a) in Richtung des freien Endbereichs einer benachbarten Ventrikularverankerungsspitze (5v) orientiert ist, die in der Umfangsrichtung nahe ist, jedoch angular bezüglich dieser Atrialverankerungsspitze (5a) versetzt ist; und umgekehrt der freie Endbereich einer Ventrikularverankerungsspitze (5v) in Richtung des freien Endbereichs einer benachbarten Atrialverankerungsspitze (5a) orientiert ist, die in der Umfangsrichtung nahe ist, jedoch angular bezüglich dieser Ventrikularverankerungsspitze versetzt ist.

9. Prothese (1) nach Anspruch 8, **dadurch gekennzeichnet, dass** jeder Basisbereich entlang einer Richtung vorsteht, die einen Winkel in der Größenordnung von 45° bezüglich der Längsachse der Prothese bildet.

10. Prothese (1) nach Anspruch 8 oder Anspruch 9, **dadurch gekennzeichnet, dass** bei jeder Spitze die Länge des Basisbereichs in der Größenordnung von zwei bis drei Mal jener des freien Endbereichs ist.

11. Prothese (1) nach einem der Ansprüche 8 bis 10, zur Behandlung einer Mitralklappe, **dadurch gekennzeichnet, dass** die Verankerungsspitzen (5a, 5v) auf folgende Weise über den Umfang der Armatur (2) der Prothese verteilt sind: eine Spitze, die an der Zone dieser Armatur angeordnet ist, die dazu ausgelegt ist, sich nach der Implantation im Bereich des anterioren Trigons zu befinden, eine Spitze, die an der Zone der Armatur (2) angeordnet ist, die dazu ausgelegt ist, sich nach der Implantation im Bereich des posterioren Trigons zu befinden, eine Spitze, die an der Zone der Armatur (2) angeordnet ist, die dazu ausgelegt ist, sich nach der Implantation im Bereich der anterioren Kommissur der Mitralklappe zu befinden, eine Spitze, die an der Zone der Armatur (2) angeordnet ist, die dazu ausgelegt ist, sich nach der Implantation im Bereich der posterioren Kommissur der Mitralklappe zu befinden, eine Spitze, die an der Zone der Armatur (2) angeordnet ist, die dazu ausgelegt ist, sich nach der Implantation in der Mitte des posterioren Rings der Mitralklappe zu befinden, sowie eine Spitze, die an der Zone der Armatur (2) angeordnet ist, die dazu ausgelegt ist, sich nach der Implantation in der Mitte des anterioren Rings der Mitralklappe zu befinden.

12. Prothese (1) nach einem der Ansprüche 1 bis 11, **dadurch gekennzeichnet, dass** die Maschenstruktur der Armatur (2) im Bereich der Atrialbereiche (2a) und ringförmigen Bereichen (2i) sowie im Bereich des Teils des Ventrikularbereichs (2v), der die Verankerungsspitzen (5v) umfasst, rautenförmige Maschen umfasst, die an ihren Ecken miteinander verbunden sind.

13. Prothese (1) nach Anspruch 12 in Kombination mit einem der Ansprüche 3 bis 7, **dadurch gekennzeichnet, dass** die Basen der Verankerungsspitzen (5a, 5v) mit den Bereichen der Maschen verbunden sind, die die entgegengesetzten Ecken dieser Maschen in der Atrial-Ventrikularrichtung der Prothese bilden, wobei diese Verankerungssitzen (5a, 5v) von diesen Maschenbereichen vorstehen.

14. Prothese (1) nach einem der Ansprüche 1 bis 13, **dadurch gekennzeichnet, dass** die Maschenstruktur der Armatur (2) im Bereich des Teils des Ventrikularbereichs (2v) ohne Verankerungsspitzen (5v) wassertropfenförmige Maschen umfasst, wobei zwei benachbarte Maschen entgegengesetzt zueinander angeordnet sind.

15. Prothese (1) nach einem der Ansprüche 1 bis 14, die dazu ausgelegt ist, eine Mitralklappe zu behandeln, **dadurch gekennzeichnet, dass** ihr Atrialbereich (2a) und ihr ringförmiger Bereich (2i) einen nicht kreisförmigen Querschnitt in Gestalt eines "D mit abgerundeten Ecken und Rändern" aufweisen.

16. Prothese (1) nach einem der Ansprüche 1 bis 15, **dadurch gekennzeichnet, dass** ihr Atrialbereich (2a) eine dichte Wand umfasst, die ihre Maschen bedeckt.

## Claims

1. Mitral or tricuspid heart valve prosthesis (1), comprising an expandable tubular frame (2) with a mesh structure and a prosthetic valve (3) mounted on the frame, said frame (2) comprising an atrial portion (2a), a ventricular portion (2v) and an annular portion (2i) situated between these atrial and ventricular portions;
- said atrial portion (2a) flares toward the outside of the prosthesis, from said annular portion (2i) toward the end of this atrial portion opposite this annular portion, and comprises, on the side of said annular portion (2i), on at least part of its circumference, anchoring spikes (5a) protruding from its outer face; and
- said ventricular portion (2v) has a substantially spherical or ovoid shape and comprises, on the side of said annular portion (2i), on at least part of its circumference, anchoring spikes (5v) protruding from its outer face°;
**characterized in that**:
- said ventricular portion (2v) has a large indentation (6) on one side, extending lengthwise from the end of this ventricular portion (2v) that is opposite said annular portion (2i) to the part (2vm) of this ventricular portion (2v) close to the annular portion (2i), this indentation (6) extending over a sector of the circumference of the prosthesis (1) of about 90 to 140 °.

2. Prosthesis (1) according to claim 1, **characterized in that** said indentation extends over a sector of the circumference of the prosthesis of about 120 °.

3. Prosthesis (1) according to claim 1 or claim 2, **characterized in that** said anchoring spikes (5a, 5v) are in the form of curved claws developing toward the outside of the prosthesis from their bases toward their pointed free ends.

4. Prosthesis (1) according to anyone of claims 1-3, **characterized in that** the anchoring spikes (5a, 5v) are present on the entire circumference of the prosthesis.

5. Prosthesis (1) according to claim 4, **characterized in that** the anchoring spikes (5a, 5v) are regularly distributed on this circumference.

6. Prosthesis (1) according to anyone of claims 1-5, **characterized in that** the anchoring spikes (5a) comprised by said atrial portion (2a) are situated across from the anchoring spikes (5v) comprised by said ventricular portion (2v).

7. Prosthesis (1) according to anyone of claims 1-6, **characterized in that** it comprises six pairs of atrial anchoring spikes (5a) and ventricular anchoring spikes (5v).

8. Prosthesis (1) according to claim 1, **characterized in that**, in the deployed state of the prosthesis:
- each anchoring spike (5a, 5v) has a substantially rectilinear base portion, protruding from the portion of the prosthesis in a direction forming an angle from 35 to 55° relative to the longitudinal axis of the prosthesis, and a free end portion that is bent relative to said base portion and oriented along a substantially circumferential direction of the prosthesis;
- the free end portion of an atrial anchoring spike (5a) is oriented toward the free end portion of an adjacent ventricular anchoring spike (5v), close in the circumferential direction but angularly offset relative to this atrial anchoring spike (5a); reciprocally, the free end portion of a ventricular anchoring spike (5v) is oriented toward the free end portion of an adjacent atrial anchoring spike (5a), close in the circumferential direction but angularly offset relative to this ventricular anchoring spike.

9. Prosthesis (1) according to claim 8, **characterized in that** each base portion protrudes along a direction forming an angle of about 45° relative to the longitudinal axis of the prosthesis.

10. Prosthesis (1) according to claim 8 or claim 9, **characterized in that**, on each spike, the length of the base portion is about two to three times that of the free end portion.

11. Prosthesis (1) according to anyone of claims 8-10, intended to treat a mitral valve, **characterized in that** the anchoring spikes (5a, 5v) are distributed as follows on the circumference of the frame (2) of the prosthesis: one spike situated on the zone of this frame intended to be found at the anterior trigone after implantation, one spike situated on the zone of the frame intended to be found at the posterior trigone after implantation, one spike situated on the zone of the frame (2) intended to be found at the anterior commissure of the mitral valve after implantation, one spike situated on the zone of the frame (2) intended to be found at the posterior commissure of the mitral valve after implantation, one spike situated on the zone of the frame (2) intended to be found at the middle of the posterior annulus of the mitral valve after implantation, and one spike situated on the zone of the frame (2) intended to be found at the middle of the anterior annulus of the mitral valve after implantation.

12. Prosthesis (1) according to anyone of claims 1-11, **characterized in that** the mesh structure of the frame (2) comprises, at said atrial and annular portions (2a, 2i), and at the ventricular portion (2v) comprising said anchoring spikes (5v), diamond-shaped meshes connected to one another by their corners.

13. Prosthesis (1) according to claim 12, linked to anyone of claims 3-7, **characterized in that** the anchoring spikes (5a, 5v) have their bases connected to the portions of the meshes that form at the opposite corners of these meshes in the atrial-ventricular direction of the prosthesis, these anchoring spikes (5a, 5v) protruding from these portions of the meshes.

14. Prosthesis (1) according to anyone of claims 1-13, **characterized in that** the mesh structure of the frame (2) comprises, at the part of the ventricular portion (2v) with no anchoring spikes (5v), teardrop-shaped mesh frames, with two adjacent meshes arranged head to tail relative to one another.

15. Prosthesis (1) according to anyone of claims 1-14, intended to treat a mitral valve, **characterized in that** its atrial portion (2a) and its annular portion (2i) have a noncircular cross-section, in the shape of a "D with rounded corners and edges".

16. Prosthesis (1) according to anyone of claims 1-15, **characterized in that** its atrial portion (2a) includes a tight wall that covers its meshes.
